# EUROPEAN PATENT APPLICATION

(11) **EP 3 241 552 A1**
(43) Date of publication of application: **08.11.2017**
(21) Application number: 17168645.4
(22) Date of filing: 28.04.2017
(51) Int. Cl.: A61K 31/22, A61K 31/366, A61K 31/40, A61K 31/405, A61K 31/4418, A61K 31/505, A61K 31/519, A61K 31/522, A61P 3/06

(54) **COMPOSITIONS AND METHODS FOR LIPID METABOLISM DISORDER**

(30) Priority: 02.05.2016 US 201662330369 P
(71) Applicant: Jansfat Biotechnology Co., Ltd., Kaohsiung City 807 (TW)
(72) Inventor: CHEN, Ing-Jun, 833 Kaohsiung City (TW)
(74) Representative: Patentanwaltskanzlei WILHELM & BECK

(57) **Abstract**

A method for inhibiting a lipid metabolism disorder of a warm-blooded animal is provided. The method includes administering a pharmaceutical composition including a phosphodiesterase-5 (PDE-5) inhibitor and a Statin analogue to the warm-blooded animal suffering from the lipid metabolism disorder.

## Description

The present invention is related to a combined therapeutic method using a pharmaceutical composition including a phosphodiesterase 5 (PDE-5) inhibitor and a Statin analogue for a warm-blooded animal suffering from the lipid metabolism disorder. In particular, the present invention is related to a pharmaceutical composition including a PDE-5 inhibitor of the first or second type and a Statin analogue to show the treatment effects.

At present, KMUP-1 has been proven to relax vascular and airway smooth muscle contractions by enhancing endothelial nitric oxide synthase (eNOS)/guanosine 3',5'-cyclic monophosphate (cGMP) pathway, including increasing the soluble guanylyl cyclase α1 (sGCα1) and protein kinase G (PKG) expression.

KMUP-1 (Kuo K.-K., et al., Journal of Lipid Research, 2015, 56(11): 2070-2084.) and Sildenafil (Das A, et al., Pharmacol Ther. 2015, 147: 12-21.) were proven to increase cGMP/protein kinase G (PKG) in the lipid metabolism, and both have been used as phosphodiesterase type 5 (PDE-5) inhibitors, but their effects on obesity were little studied. An increase of cGMP can promote the expansion and reduction of white adipose tissues, unlike visceral epididymal fat pads. Indeed, eNOS and HSL (hormone-sensitive lipase) in obesity are related to decreased subcutaneous adipose tissue lipolysis, the inhibition of NOS resulted in increased lipolysis in white tissues, and the addition of nitric oxide (NO) caused the inhibition of lipolysis.

In accordance with one aspect of the present invention, a method for treating a lipid metabolism disorder is disclosed. The method includes a step of administering a therapeutically effective amount of an active agent being one selected from the group consisting of a PDE-5 inhibitor of the first type and second type or a Statin analogue to a warm-blooded animal suffering from the disease being one selected from the group consisting of liver disease, non-alcoholic fatty liver disease, hyperadiposity, dyslipidemia, hepatic steaotosis, high-fat-diet-induced lipid accumulation, high-fat-diet-induced obesity, insulin resistance, high-fat-diet-induced lipid accumulation combined with a symptom of one of inflammation and liver damage, and any combination thereof.

In accordance with another aspect of the present invention, a pharmaceutical composition is disclosed, which includes a PDE-5 inhibitor or a pharmaceutical acceptable salt thereof, and a Statin analogue, and/or an acceptable carrier, diluent or excipient.

A further aspect for the composition is to administer the therapeutically effective amounts of a PDE-5 inhibitor and a Statin analogue (an active agent) to a warm-blooded animal in need thereof.

The present invention will now be described more specifically with reference to the following embodiments. It is to be noted that the following descriptions of preferred embodiments of this invention are presented herein for the purposes of illustration and description only; they are not intended to be exhaustive or to be limited to the precise form disclosed.

Phosphodiesterases are a diverse family of enzymes that hydrolyse cyclic nucleotides and thus play a key role in regulating intracellular levels of the second messengers cyclic-3',5'-adenosine monophosphate (cAMP) and cyclic-3',5'-guanosine monophosphate (cGMP), and hence represent cell function. At present, at least 11, and possibly 12 subfamilies have been identified that catalyze the hydrolysis of cAMP, cGMP or both.

Several investigations have demonstrated that increased cGMP signaling might be an important strategy for reducing the progression of diabetes through multiple pathways. Concerning glycemic control, even if an increase in intracellular calcium is the principal signal that activates insulin exocytosis, cGMP may also participate through distinct signals and potentiate the stimulation of glucose.

In parallel, some *in vitro* studies have shown that cGMP may enhance insulin sensitivity in target organs (muscle and adipocytes) by stimulating glucose transporter type 4 (GLUT4) recruitment into the plasma membrane. In addition, because NO/cGMP signaling is fundamental to vascular protection, the increment of this pathway may be an attractive strategy to attenuate endothelial dysfunction development in diabetic complications, which is a major cause of disability and death in patients with diabetes mellitus.

Pharmacological strategies to increase cGMP signaling may be achieved through two main routes: (1) direct activation of guanylate cyclase directly by augmentation of NO; and/or (2) decreasing cGMP hydrolysis through PDE5 inhibitor, which is currently considered an important tool to treat endothelial dysfunction in diabetes mellitus.

PDE5 is expressed in many tissues (e.g. heart, lung, pancreas, and penis) and plays a specific role in hydrolyzing cyclic guanosine monophosphate (cGMP). In adipocytes, cGMP regulates crucial functions by activating cGMP-dependent protein kinase G (PKG). Therefore, it is extremely important to preserve its physiological health in order to avoid local and systemic disorders. Experiments available in literature show the importance of NO/cGMP/PKG pathway in adipocyte biology. Phosphodiesterase 5 (PDE5) is an enzyme responsible for cGMP inactivation, and the use of its inhibitors can be an alternative in the search of a more balanced adipose tissue.

Numerous nonselective phosphodiesterase (PDE) isoforms are known with xanthine derivative, for example, 3-isobutyl-1-methylxanthine, caffeine, theophylline and theobromine. Interestingly, PDE5 inhibitors were recently shown to affect adipose differentiation and aromatase function. The expressive therapeutic benefit of PDE5 inhibitors for the active agent use, in the present invention that can be grouped on the basis of their structural similarity, two types are distributed as follows: 1) piperazinyl analogues and piperazinyl analogue complexs, 2) heterocyclic derivatives, for example, pyrrolidinyl, purine, quinazolin, and pyridinyl, and the fused with nitrogen-containing heterocyclic derivatives, for example, pyrimido[5,4-d]pyrimidin, pyrazinopyrido[3,4-b]indole, triazolo[4,5-d]pyrimidin, and glycoside derivative.

In a particular embodiment, the term "second type PDE5 inhibitor" as used herein includes, but is not limited to, Avanafil, Benzamidenafil, Dasantafil, Dipyridamole, E4021, Icariin, Rolipram, Piclamilast, Tadalafil and Zaprinast. The compound Avanafil has the chemical name 4-[(3-chloro-4-methoxyphenyl)amino]-2-[(2S)-2-(hydroxymethyl)-1-pyrrolidi nyl]-N-(2-pyrimidinylmethyl)-5-pyrimidin carboxamide. The compound Benzamidenafil has the chemical name N-(3,4-dimethoxy-benzyl)-2-((2-hydroxy-1-methylethyl)amino)-5-nitrobenzamide. The compound Dasantafil has the chemical name 7-[(3-bromo-4-methoxyphenyl)methyl]-1-ethyl-8-[(2-hydroxycyclopentyl)amino] -3 -(2-hydroxyethyl)purine-2,6-dione. The compound Dipyridamole has the chemical name 2-[[2-[bis(2-hydroxyethyl)amino]-4,8-di(piperidin-1-yl)pyrimido[5,4-d]pyrimidin-6-yl]-(2-hydroxyethyl)amino]ethanol. The compound E4021 has the chemical name sodium; 1-[4-(1,3-benzodioxol-5-ylmethylamino)-6-chloroquinazolin-2-yl]pipe ridine-4-carboxylate. The compound Icariin has the chemical name 3,5,7-three hydroxy-4'-methoxy-8-prenyl flavonoids-3-O-α-L-topiramate Nom rhamnose-7-0-D-glucopyranoside. The compound Rolipram has the chemical name 4-[3-(cyclopentyloxy)-4-methoxyphenyl] -2-pyrrolidinone. The compound Piclamilast has the chemical name 3-(cyclopentyloxy)-*N*-(3,5-dichloropyridin-4-yl)-4-methoxybenzamide. The compound Tadalafil has the chemical name (6R-*trans*)-6-(1,3-benzodioxol-5-yl)-2-methyl-2,3,6,7,12,12a-hexahydropyrazino[1',2':1,6]pyrido[3,4-b]indole-1,4-dione. The compound Zaprinast has the chemical name 5-(2-propoxyphenyl)-2,3-dihydrotriazolo[4,5-d]pyrimidin-7-one.

In accordance with one aspect of the present invention, the term "the first type PDE5 inhibitor" as used herein includes, but is not limited to, piperazinyl analogue and piperazinyl analogue complex. Both piperazinyl analogue and piperazinyl analogue complex are classified into two subcategories individually, wherein piperazinyl analogue includes KMUPs derivatives and Sildenafil analogues, and piperazinyl analogue complex includes KMUPs dereviative, KMUPs derivative complex and Sildenafil analogue complex. KMUPs derivative complex can be designated by the general formulae KMUPs-RX (formula I), or KMUPs-RX-RX (formula II), and Sildenafil analogue complex is known as the general formula Sildenafil analogue-RX (formulae IV and V). The RX group is a carboxylic group donor, which bonds to one of the active agents which are a KMUPs derivative compound (formula III) or a Sildenafil analogue (formula VI), which can produce KMUPs derivative complex or Sildenafil analogue complex.

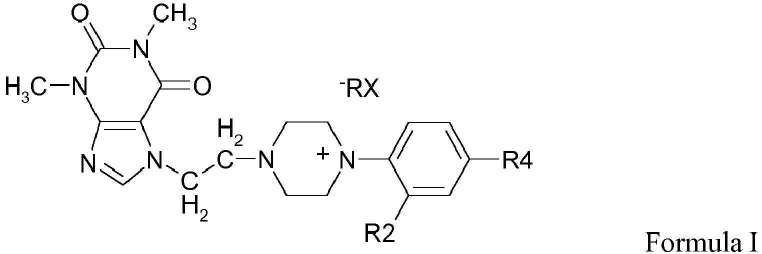

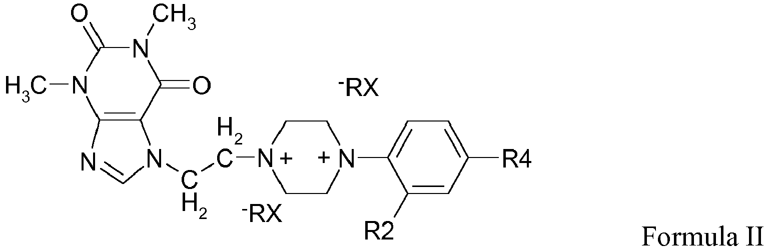

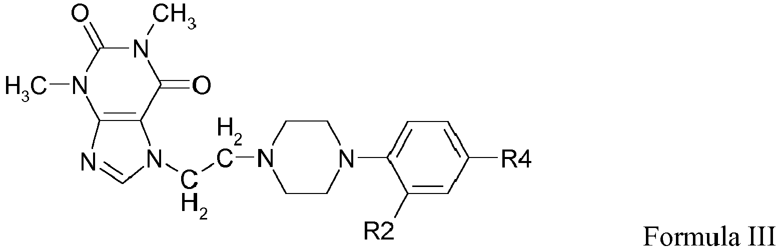

Preferably, both the general formula I and the general formula II represent the KMUPs derivative complex, in which the monomer KMUPs-RX (formula I) formed from one molecule of RX group is contacted and the KMUPs-RX-RX (formula II) is the dimer contacting two molecules of RX group. The substituent group R₂ and R₄ of KMUPs derivative (Formula III) are each selected independently from the group consisting of C1-C5 alkoxy group, hydrogen atom, nitro group, and halogen atom. The above-mentioned halogen refers to a fluorine atom, chlorine atom, bromine atom and iodine atom.

The term "KMUPs derivative complex" as used herein refers to one selected from the group consisting of KMUP-1, KMUP-2, KMUP-3, KMUP-4 and its pharmaceutical acceptable salts. Preferably, the pharmaceutical composition further includes at least one of a pharmaceutically acceptable carrier and an excipient.

Preferably, in one embodiment, KMUP-1, where R₂ is a chlorine atom and R₄ is a hydrogen atom, has the general chemical name 7-[2-[4-(2-chlorophenyl)piperazinyl]ethyl]-1,3-dimethylxanthine. KMUP-2, where R₂ is a methoxy group and R₄ is a hydrogen atom, has the chemical name 7-[2-[4-(2-methoxybenzene)piperazinyl]ethyl]-1,3-dimethylxanthine. KMUP-3, where R₂ is hydrogen and R₄ is a nitro group, has the chemical name 7-[2-[4-(4-nitrophenyl)piperazinyl]ethyl]-1,3-dimethylxanthine. KMUP-4, where R₂ is a nitro group and R₄ is a hydrogen atom, has the chemical name 7-[2-[4-(2-nitrophenyl)piperazinyl]ethyl]-1,3-dimethylxanthine.

In a particular embodiment, the term "RX group" as used herein refers to one selected from the group consisting of D-ascorbic acid, L-ascorbic acid, DL-ascorbic acid, oleic acid, phosphoric acid, citric acid, nicotinic acid, sodium carboxymethylcellulose (sodium CMC), hyaluronic acid, polyacrylic acid (PAA), polymethylmethacrylates (PMMA), Eudragit®, dextran sulfate, heparan sulfate, polylactic acid or polylactide (PLA), polylactic acid sodium (PLA sodium), polyglycolic acid sodium (PGCA sodium), poly-γ-polyglutamic acid sodium (γ-PGA sodium), γ-polyglutamic acid (γ-PGA), alginate-poly-1-lysine-alginate (APA) and poly-γ-polyglutamic acid derivative.

In a particular embodiment, the pharmaceutically acceptable salts of KMUP-1, KMUP-2, KMUP-3 and KMUP-4 include, but are not limited to citric acid, nicotinic acid and hydrochloride. On the other hand, it has been found that in addition to the known Sildenafil analogue compounds, the salts of the analogues are not limited to citrate salt, disodium salt and hydrochloride salt.

In accordance with an aspect of the present invention, the term "Sildenafil analogue compound" has the chemical structure of a heterocyclic ring. The Sildenafil analogue is represented by the chemical structure of general formula VI. The substituent RB group includes, but is not limited to the substituted heterocyclic group, for example the pyrazolopyrimidinone group, pyrazinopyridoindole group, imidazotriazinone group and pyrrolidin group. The six-membered ring on the heterocyclic backbone usually has an oxygen atom or a sulfur atom linked by a double bond between the carbons on the ring, and an oxygen atom of the oxyl-thiazol-N-methylnitrous amide linked by a single bond between the carbons on the ring. The substituent RS is bonded to a sulfonylphenyl group or acetyl phenylgroup based on the formula. The substituent R3 includes an alkyl group, and R7 includes a substituted or unsubstituted piperidine group, pyrrolidin group, hydrogen atom, N-alkyl group, alkoxyl group, alkanol group.

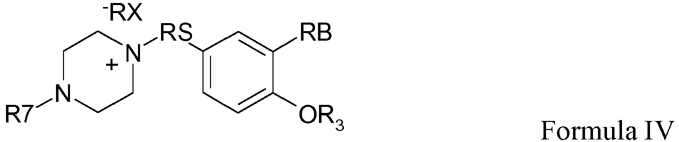

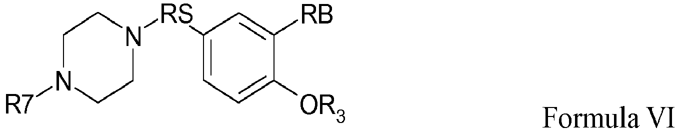

Sildenafil analogue compounds have been approved by the pharmaceutical, medical and health-related governmental authorites in some countries, such as the United States Food and Drug Administration (U.S. FDA), China, Europe, Australia, United Kingdom, Canada and Japan.

A bulk material of Sildenafil analogues has been developed. Therefore, the term "Sildenafil analogue" as used herein includes but is not limited to the following: Acetidenafil, Aildenafil, Avanafil, Benzyl-Sildenafil, Carbodenafil, Cinnamyldenafil, Cyclopenylnafil, Descarbon-Sildenafil, N-Desmethyl-Sildenafil, Desmethyl Carbodenafil, Dimethyl-Acetidenafil, Dioxy-Acetidenafil, Dithiodesmethyl Carbodenafil, Gendenafil, Gisadenafil, Hydroxy-Acetidenafil, Hydroxy Chlorodenafil, Hydroxyhomo-Sildenafil, Hydroxythiohomo-Sildenafil, Homo-Sildenafil, Isopiperazinonafil, Lodenafil Carbonate, Mirodenafil, Nitrodenafil, Nor-Acetidenafil, Normeo-Sildenafil, Piperildino-Acetidenafil, Piperazinonafil, Propoxyphentyl-Aildenafil, Propoxyphentyl-Sildenafil, Propoxyphentylthio-Aildenafil, SulfohomoSildenafil, ThioAildenafil, ThioSildenafil, Udenafil and Vardenafil. As used herein, Sildenafil analogues include, but are not limited to its free base, or a pharmaceutically acceptable salt, solvate, solvate of such a salt or a prodrug thereof.

In a preferred embodiment, the term "Sildenafil analogue" as described herein includes, but is not limited to Acetidenafil, Avanafil, DesmethylSildenafil, Gisadenafil, HomoSildenafil, HydroxyhomoSildenafil, Mirodenafil, Lodenafil Carbonate, Sildenafil, Udenafil, and Vardenafil. The compound Acetidenafil has the chemical name 5-{2-ethoxy-5-[2-(4-ethylpiperazin-1-yl)acetyl]phenyl}-1-methyl-3-propyl-4H-pyrazolo[4,3-d]pyri midin-7-one. The compound Avanafil has the chemical name 4-[(3-Chloro-4-methoxyphenyl)methylamino]-2-[(2S)-2-(hydroxymethyl)pyrr olidin-1-yl]-N-(pyrimidin-2-ylmethyl)pyrimidine-5-carboxamide. The compound DesmethylSildenafil has the chemical name 5-[2-ethoxy-5-piperazin-1-ylsulfonylphenyl]-1-methyl-3-propyl-4H-pyrazolo[4,3-d]pyrimidi n-7-one. The compound Gisadenafil has the chemical name 5-[2-Ethoxy-5-(4-ethyl-piperazin-1-yl)sulfonylpyridin-3-yl]-3-ethyl-2-(2-methoxyethyl)-4H-p yrazolo[4,3-d]pyrimidin-7-one. The compound HomoSildenafil has the chemical name 5-[2-ethoxy-5-(4-ethylpiperazin-1-yl)sulfonylphenyl]-1-methyl-3-propyl-4H-pyrazolo[4,3-d]pyrimidin-7-one. The compound HydroxyhomoSildenafil has the chemical name 5-[2-ethoxy-5-[4-(2-hydroxy ethyl)piperazin-1-yl]sulfonylphenyl]-1-methyl-3-propyl-4H-pyrazolo[4,3-d]py rimidin-7-one. The compound Mirodenafil has the chemical name 5-ethyl-2-{5-[4-(2-hydroxyethyl)piperazin-1-yl]sulfonyl-2-propoxyphenyl}-7-propyl-1H-pyrrolo[3,2-d]pyrimidin-4-one. The compound Lodenafil Carbonate has the chemical name bis[2-[4-[4-ethoxy-3-(1-methyl-7-oxo-3-propyl-4H-pyrazolo[4,3-d]pyrimidin-5-yl)phenyl]sulfonylpiperazin-1-yl]ethyl] carbonate. The compound Sildenafil has the chemical name 5-[2-ethoxy-5-(4-methylpiperazin-1-yl)-sulphonylphenyl]-1-methyl-3-propyl-4 H-pyrazolo[4,3-d]pyrimidin-7-one, or 1-[3-(4,7-dihydro-1-methyl-7-oxo-3-propyl-1H-pyrazolo[4,3-d]pyrimidin-5-yl)-4-ethoxyphenyl]sulfonyl] -4-methyl piperazine. The compound Udenafil has the chemical name 3-(l-methyl-7-oxo-3-propyl-4H-pyrazolo[4,3-d]pyrimidin-5-yl)-N-[2-(1-meth ylpyrrolidin-2-yl)ethyl]-4-propoxybenzene sulfonamide. The compound Vardenafil has the chemical name 2-[2-ethoxy-5-(4-ethylpiperazin-1-yl)-sulfonylphenyl]-5-methyl-7-propyl-1H-imidazo[5,1-f][1,2,4]triazin-4-one.

The term "Statins analogue" as used herein refers to commercially available Statin derivative drugs. In some embodiments, the Statins analogues include but are not limited to, Atorvastatin, Cerivastatin, Fluvastatin, Lovastatin, Mevastatin, Pravastatin, Rosuvastatin, Simvastatin, and Pravastatin acid, and their pharmaceutically acceptable salts.

In accordance with a further aspect of the present invention, depending on the desired clinical use and effect, an adaptable administration method for the pharmaceutical composition consisting of a PDE-5 inhibitor and a Statin analogues is disclosed in the present invention, and the administration includes one selected from an oral administration, an intravenous injection, a subcutaneous injection, an intraperitoneal injection, an intramuscular injection and a sublingual administration.

According to a further aspect of the present invention, a method for generating a therapeutic effect for a lipid metabolism disorder in a warm-blooded animal (such as a human, goat, lamb, sheep, pig, cow, chicken, duck) in need of such treatment is disclosed in the present invention, and the treatment is performed by administering said animal an effective amount of one of the active agents being selected from the group consisting of a PDE-5 inhibitor and a Statin analogue, or a pharmaceutically acceptable salt, solvate, solvate of such prodrug thereof.

The term "carrier" as used herein refers to an excipient that may be formulated with the active agent to produce a composition. The sterile composition for injection can be dissolved or suspended in a non-toxic intravenous injection diluent or a solvent such as 1,3-butanediol. Among these carriers, the acceptable carrier may be mannitol or water. In addition, the fixing oil, the synthetic glycerol ester or the di-glycerol ester are commonly used solvent. A fatty acid such as oleic acid, olive oil, castor oil and glycerol ester derivatives thereof, especially the oxy-acetylated type, may serve as the oil for preparing the injection and as the naturally pharmaceutically acceptable oil. Such oil solution or suspension may include the long chain alcohol diluents or the dispersing agents, the carboxylmethyl cellulose or the similar dispersing agents. Other carriers are common surfactants such as Tween and Spans or other similar emulsions, or a pharmaceutically acceptable solid, liquid or other bio-avaliable enhancement agent used for developing a formulation that is used in the pharmaceutical industry.

For the orally administered formulation, carrier particles are used to improve the flowablility of drug particles and the accuracy of the dose and minimize the variability of the dosedrug formulation so as to facilitate the observation of the drug formulation, and the stability of the manufacturing process. The composition for the oral administration may be of any acceptable oral formulation, which includes a capsule, tablet, pill, emulsion, aqueous suspension, dispersing agent or solvent. Taking a tablet as an example, the carrier may be basic additives such as lactose, corn starch, lubricant, and magnesium stearate. The diluents used in the capsule include lactose and dried corn starch. To prepare the aqueous suspension or the emulsion formulation, the active ingredient is suspended or dissolved in an oil interface in combination with an emulsion or the suspending agent, and an appropriate amount of a sweetening agent, flavor or pigment is added as needed.

The compound of the present invention can also be prepared as the injection administration that is administered intravenously, as well as subcutaneously, parentally, into muscles, or by intra-articular, intracranial, intra-articular fluid and intra-spinal injections, aortic injection, sterna injection, intra-lesion injection or other appropriate administration.

In an embodiment of the present invention, a combination therapy is adapted for treating and/or preventing a lipid metabolism disorder. The compositions are formulated and administered in the manner detailed herein. The active agent being one selected from the group consisting of PDE-5 inhibitors of the first type and the second type may be effectively used in combination with a Statin analogue depending on the desired targeted therapy. The combination therapy includes administering a single pharmaceutical dosage composition which contains a PDE-5 inhibitor and a Statin analogue and one or more pharmaceutical excipients, and each ingredient has its pharmaceutical dosage and formulation.

For example, the compounds selected from the group consisting of PDE-5 inhibitors of the first type and the second type and a Statin analogue can be administered together to a patient in a single oral dosage composition such as the tablet or the capsule, or each compound can be administered in an individual oral dosage formulation. Where individual dosage formulations of different active agents are used, they can be administered in another administration type at essentially the same time. An example of combination treatment may be by any suitable administration route including oral (including buccal and sublingual), rectal, nasal, vaginal, and parenteral (including subcutaneous, intramuscular, intravenous and intradermal). Topical administrations include, but are not limited to, spray, plaster, mist, aerosol, solution, lotion, gel, cream, ointment, paste, unguent, emulsion and suspension, with oral or parenteral being preferred. The preferred route may vary with the condition and age of the recipient.

While it is possible for the administered ingredients to be administered alone, it is preferable to include them as part of a pharmaceutical formulation. The formulations of the present invention include the administered two ingredients, as defined above, together with one or more acceptable carriers. The carrier(s) must be "pharmaceuticaly acceptable" in the sense of being compatible with the other ingredients of the formulation and not deleterious to the recipient.

According to the above-mentioned aspect of the present invention, the various active agents may include additional compounds according to the invention, or one or more other pharmaceutically active agents. In some preferable embodiments, the inventive compositions contain the active agents, including the inventive combination of therapeutic agents, in an amount effective to treat an indication of symptoms.

A therapeutic substance being distributed is suitably administered in any way in which at least some (preferably about 1 weight (wt.) % to about 100 wt. %) of the substance reaches the bloodstream of the organism. Thus, the substance can be administered enterally (via the alimentary canal) or parenterally (via any route other than the alimentary canal, such as the intravenous injection, subcutaneous injection, intramuscular injection, and inhalation percutaneous application, etc.).

A pharmaceutical composition is disclosed in the present invention, includes the active agent being consisting of a PDE-5 inhibitor and a Statin analogue which can increase cyclic guanosine monophosphate (cGMP) to adipogenesis or lipoysis to enable the skilled person in the art to use the role of phosphorylated hormone-sensitive lipase/adipose triglyceride lipase (p-HSL/ATGL) for treating a lipid metabolism disorder.

A pharmaceutical composition is disclosed in the present invention, in which the active agent is selected from the group consisting of PDE 5 inhibitors of the first type and the second type and a Statin analogue for inhibiting lipid accumulation, reducing steatohepatitis leading to inflammation, and the pharmaceutical composition is related to the treatment and/or prevention of the lipid metabolism disorder.

Adipose tissue, acting as an endocrine organ, is responsible for the release of multiple cytokines, which have the most diverse metabolic functions. Metabolism is the process that the organism digests the nutrients (proteins, carbohydrates and fats) to convert into energy. The organism can store energy in the liver, muscles and body fat. The organism's cells can provide energy to synthesize new organic materials via the biological processes.

White adipocytes specialize and differentiate into spherical cells with a great capacity to store triacylglycerol for subsequent release of fatty acids under the conditions of high metabolic demand (i.e. exercise) or negative energy balance (i.e. food restriction). Similar to the endocrine role of white adipocytes, brown adipose tissue also synthesizes and secretes numerous factors.

Insulin-mediated deactivation of lipolysis is associated with transcriptional downregulation of ATGL and HSL expression. Additionally, insulin signaling results in phosphorylation and activation of various phosphodiesterase (PDE) isoforms by PKB/Akt leading to PDE-catalyzed hydrolysis of cAMP which in turn results in reduced activation of PKA.

Disturbances in fatty acid metabolism in adipose tissue, liver, skeletal muscle, gut and pancreas play an important role in the development of insulin resistance, impaired glucose metabolism and type 2 diabetes mellitus. During the endocrine dysfunction, the reduced capacity to store and transform lipids has more of an impact on the development of insulin resistance, and metabolism disorder. Metabolic disease includes all of the diseases or disorders that disrupt normal metabolism process. In particular, metabolic disorders include type 1 or type 2 diabetes, and related disorders such as obesity, impaired glucose tolerance, hyperinsulinemia, dyslipidemia and insulin resistance.

In recent years, one experiment on high-fat diet-induced obese mice showed loss of macrophage inflammatory protein-1α, a chemokine and abrogated M2 macrophage-polarizing and insulin-sensitizing effects (Zhuge F., et al., Diabetes, 2016. 65(10): 2966-2979.).

Other reports from human and animal studies also suggest that cytokines and chemokines produced by macrophages generate local and systemic inflammation and this condition leads to pancreatic β-cell dysfunction and insulin resistance in liver, adipose and skeletal muscle tissues. Macrophages contribute to type 2 Diabetes mellitus complications through cell-cell interactions and the release of pro-inflammatory cytokines, chemokines, and proteases to induce inflammatory cell recruitment, cell apoptosis, angiogenesis, and matrix protein remodeling (Meshkani R., et al., Clin. Chim. Acta, 2016, 26: 462:77-89.).

Those studies demonstrate that the effects of obesity on chronic and intermittent hypoxia in white adipose tissue and the resulting effects on gene expression and cell metabolism. Particularly chronic inflammation in adipose tissue seems to play an important role in the development of obesity-related insulin resistance.

G-protein-coupled receptors (GPCRs) are the most common potential pharmacological targets but few reports indicate that GPCRs antagonists inhibit the adipogenesis of adipose tissues. GPCRs-mediated activation of major kinases, including mitogen-activated protein kinase (MAPK), extracellular signal-regulated protein kinases (ERK) and Mitogen-activated protein kinase 1 (MEK1), is likely to become important for the identification of GPCR antagonists targeting adipogenesis or lipolysis.

Statins worked best to reduce plasma low-density lipoprotein cholesterol (LDL-C) and high-sensitivity c-reactive protein levels. Niacin is used as a hypolipidaemic drug, which mediates lipolysis in adipose tissue through its G-protein coupled receptor. However, recent outcome trials failed to show niacin's benefit on top of statin therapy in lipid-targeted approaches to reduce major cardiovascular events in high-risk patients.

Long term GPCRs antagonist activity of KMUP-1 during lipolysis of hepatocytes/adipocytes is useful to protect from chronic over-stimulation by catecholamine seen in obesity, in contrast to GPCR agonist unfavourably used for anti-obesity via decreasing appetite due to stimulation of central nervous systems. In this situtation, KMUP-1 increases PKA/PKG/HSL to stimulate the lipolysis of adipocytes/hepatocytes via inhibition of phosphodiesterase (PDE).

Elevated circulating free fatty acid levels, in part related to diminished suppression of adipose tissue lipolysis by insulin, results in increased delivery of free fatty acids to the liver. The synthesis of excess triglyceride in the liver is driven by this supply of fatty acids, and the accumulation of excess liver fat is further exacerbated by impaired hepatic fatty acid oxidation secondary to insulin resistance.

When glucose levels are elevated in the context of pre-diabetes or overt diabetes, this provides further substrate for triglyceride synthesis. Additionally, impaired very low density lipoprotein (VLDL) secretion, which commonly occurs with insulin resistance, further contributes to hepatic fat accumulation. Insulin resistance is not only a factor in obesity and diabetes, but also may be an underlying mechanism for non-alcoholic fatty liver disease even in non-obese individuals without diabetes, as noted in a euglycemic insulin clamp study.

Hepatic steaotosis may be induced by hyperadiposity via an HFD, hyperadiposity in the liver involves lipid accumulation combined with inflammation, which may lead to further liver damage. Steatohepatitis is usually accompanied by oxidative stress via reactive oxygen species (ROS) after long-term administration of an HFD. HFD-induced obesity causes oxidative stress via ROS, indirectly increasing MAPK and ERK expression via hepatocytes. We sought to suppress ROS and inhibit inflammatory tumor necrosis factor-alpha (TNF-α) and matrix metallopeptidase-9 (MMP-9) in adipocytes surrounded by macrophages.

Metabolic syndrome represents a cluster of related metabolic abnormalities, including central obesity, hypertension, dyslipidemia, hyperglycemia, and insulin resistance, with central obesity and insulin resistance in particular recognized as causative factors. These metabolic derangements present significant risk factors for cardiovascular disease, which is commonly recognized as the primary clinical outcome. This cluster of problems puts people at a higher risk of cardiovascular disease and type 2 diabetes.

As body fat stores expand with calorie excess and progressive obesity, alterations in lipid metabolism together with inflammation in adipose tissue and ectopic sites of fat deposition lead to insulin resistance predominantly secondary to post-receptor abnormalities in insulin signaling pathways.

Adipose tissue inflammation in obesity is characterized by macrophage types affected by classically activated macrophages (M1,CAM)/alternatively activated macrophages (M2, AAMs) switching, and immunostaining for macrophages was massive in most HFD-induced liver inflammation.

Therapies to raise the levels of high-density lipoprotein (HDL) and lower the low-density of lipoprotein (LDL) levels are thought to exert atheroprotective effects via activation of eNOS, elevation of peroxisome proliferator activated receptor γ (PPARγ) and inhibition of scavenger receptor class B type I (SR-B 1).

Application of mevalonate to liver cells results in biosynthesis of the isoprenoid compounds farnesyl pyrophosphate (FPP) and geranylgeranyl pyrophosphate (GGPP), levels of which are reduced by HMG CoA reductase inhibitor statins, and GGPP-derived activation of RhoA/ROCK II and the following upregulation of PPARγ are involved in increasing high density lipoprotein (HDL). ATP-binding cassette transporter ABCA1 (member 1 of human transporter sub-family ABCA), and apolipoprotein A-I (ApoA-I) are involved in the regulation of cholesterol efflux and are the major protein components of HDL.

Cellular cholesterol homeostasis is accomplished, in part, by PPARs and Liver X receptor (LXRα). Statin-induced inactivation of RhoA/ROCK contributes to activation of LXRα/PPARs and their pleiotropic effects. Isoprenoid intermediates affect PPARs and LXRα activation. Activation of isoprenoid produces FPP and GGPP, which inhibit ABCA1 directly through antagonism of LXRα and indirectly through RhoA by activation of geranylgeranylation. PPARγ is expressed in fat storage and associated inflammation.

### Effects on weight gain, food intake and lipid profiles in serum

Table 1 shows the 8-week body-weight gain of animals fed with a standard diet (STD) or a high-fat diet (HFD). Consumption of HFD for 8 weeks significantly increased body-weight gain compared to the STD group (*p* < 0.05). KMUP-1 HCl (2.5, 5 mg/kg p.o.) and simvastatin supplementation (5 mg/kg p.o.) reduced body-weight gain, compared to the control HFD group (*p*<0.05). The reduction of body-weight gain and remained triacylglycerol (TG) by simvastatin, was less and higher than that of KMUP-1, respectively.

HFD caused dramatic increases in serum TG, total cholesterol, LDL cholesterol and insulin compared with the STD group. HFD-induced hypercholesterolemia was significantly improved by KMUP-1, KMUP-1-DL-ascorbic acid + Simva (KMUP-1-DL-ascorbic acid + Simva), KMUP-1-CMC + Simva supplementation. Particularly, the insulin level has shown improvement from the treatment of these drugs. In addition, HDL cholesterol level was significantly increased by KMUP-1-DL-ascorbic acid + Simva, and KMUP-1-CMC + Simva. When the food intake in animals fed a HFD after maturity (8 weeks) slowed down, the period of feeding was prolonged to 14 weeks. Some factors that affected the food intake by the animals remained unclear.

**Table 1 The effect of KMUP-1 and Sildenafil on lipid levels, body weight and food intake of mice fed with HFD**

| | STD | HFD | HFD + target drug (mg/kg) | | |
|---|---|---|---|---|---|
| | | | KMUP-1 | | SIDAF |
| | | | 2.5 | 5 | 5 |
| TG in serum | 107.2±6.1 | 166.8±5.3* | 74.5±5.1** | 72.7±4.7** | 82.7±6.3** |
| TG of liver | 42.3±3.2 | 78.6±5.3^{##} | 28.4±3.6** | 26.12±3.1** | 34.5±2.** |
| Tot. chol. | 78.7±1.9 | 206.8±13.4^{##} | 133.0±5.1* | 125.5±9.8* | 133.7±4.3* |
| HDL | 60.4±1.6 | 68.4±3.5^{#} | 103.6±4.2* | 118.3±5.7* | 103.2±2.5* |
| LDL | 6.0±0.3 | 31.3±7.0^{##} | 14.2±1.4* | 14.2±2.2* | 15.3±1.3* |
| intake | 4.0±0.2 | 2.4±0.1^{#} | 2.3±0.1* | 2.2±0.1* | 2.1±0.1* |
| initial BW | 21.1±0.3 | 22.1±0.8 | 22.0±0.3 | 21.2±0.7 | 21.1±0.8 |
| final BW | 24.1±0.5 | 29.1±0.9^{#} | 25.7±0.7* | 24.3±0.5* | 23.9±0.9* |
| BW gain | 3.0±0.4 | 6.9±0.7^{#} | 3.7±0.5* | 3.1±0.6* | 2.8±0.3* |

| | | | | | |
|---|---|---|---|---|---|
| (note) STD = standard diet, HFD = high-fat diet, Simva = Simvastatin, KMUP-1 = KMUP-1 HCl SIDAF = Sildenafil = Sildenafil citrate TG = Triacylglycerol (mg/dl), chol. = cholesterol (mg/dl), Tot. chol. = total cholesterol (mg/dl), HDL = HDL cholesterol (mg/dl), LDL = LDL cholesterol (mg/dl), intake = food intake (g/day), initial BW = initial Body weight (g), final BW = Body weight (g), BW gain = Body weight gain (g/day), | | | | | |

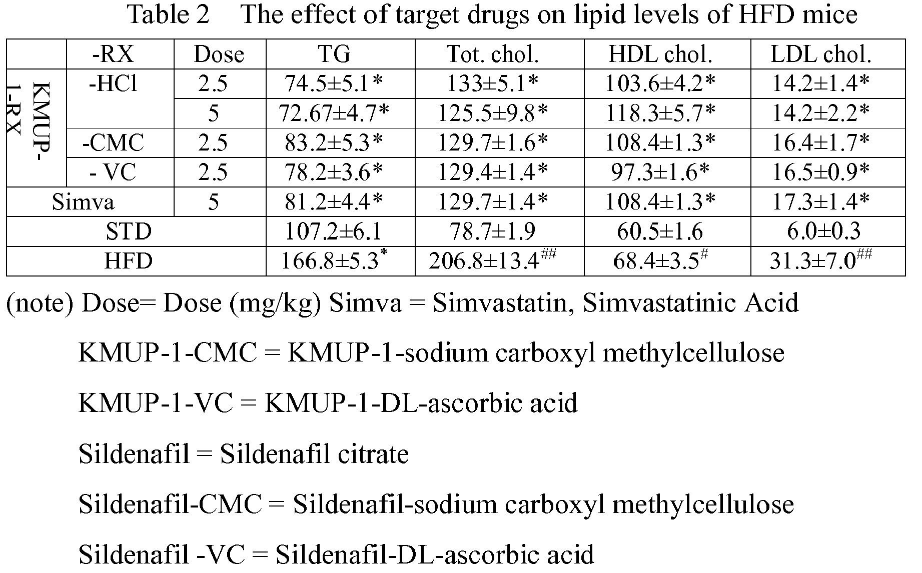

KMUP-1-CMC = KMUP-1-sodium carboxyl methylcellulose KMUP-1-VC = KMUP-1-DL-ascorbic acid Sildenafil = Sildenafil citrate Sildenafil-CMC = Sildenafil-sodium carboxyl methylcellulose Sildenafil -VC = Sildenafil-DL-ascorbic acid

### Weight changes and gross morphology of livers

The drinking of KMUP-1 HCl (2.5 mg/200 ml water) by mice fed a HFD decreased the body weight in both the protection and treatment groups. Fatty tissues were characteristically found on the surface of liver fed with HFD. Fatty liver was markedly decreased in the protective group and this effect was more prominent than in the treatment group.

### HFD-induced SR-B1 expression in the liver

Exploring KMUP-1 on increased HDL, drinking KMUP-1 was observed to inhibit HFD-induced hepatic SR-B1 expression (Table 3) and promote PKA/PKG expression in both the protection and treatment groups.

**Table 3 The effect of HFD-induced hepatic SR-B1 expression**

| | Groups | | |
|---|---|---|---|
| Expression | Control | Protection | Treatment |
| SR-B1 | 100% | 30% | 59% |
| PKA/PKG | 550% | 250% | 100% |

### H&E staining of liver

The gross morphology of inflammatory liver isolated from a fatty mouse liver fed with HFD for 14 weeks, which was rich in white fat tissues compared to the red-brown liver found in the protection group supplemented with KMUP-1+HFD for 14 weeks.

The Hematoxylin and Eosin (H&E) staining of liver sections in which macrovesicular fat globules cleaned with organic solvent were found in liver of mice treated with HFD for 14 weeks/last 6 weeks in the treatment or protection group. Obese mice supplemented with HFD at week 8 were treated with oral KMUP-1 (2.5 mg/kg) for 6 weeks from week 8 to week 14. Mallory's hyaline bodies and fat globules were found. Pretreatment with oral KMUP-1 (2.5 mg/kg) during the last 6 weeks/14 weeks reduced fat globules and Mallory's hyaline bodies, which almost disappeared.

### IHC staining of HSL/p-HSL/ATGL in steatohepatitis

The liver section from the HFD mice was dark brown, indicating large amounts of inflammatory proteins or enzymes, including inflammatory MMP-9/TNF-α, inactive HSL/p-HSL/ATGL, hyperadiposity via multiple white oil globulets and ROS production in an obese liver.

The deep brown color of the antibody response to TNF-α were shaded in both the treatment and protection groups, in which the oil globulets almost disappeared, but the color response could not be reduced substantially, indicating an incomplete anti-inflammatory effect. The deep brown color of the anti-body response to MMP-9 was also shaded by the treatment and almost disappeared in the protection group. Note Table 4 shows the oil globulet number and changes of oil globulet diameter were sharply reduced in the treatment and protection groups. Even in the negative control, without the addition of an antibody, the oil globulets in the HFD group decreased or disappeared after KMUP-1 treatment.

**Table 4 The amounts and diameter of liver fat globules**

| | Groups | | |
|---|---|---|---|
| Amounts | Control | Protection | Treatment |
| TNF-α | 57 | 18 | 19 |
| MMP-9 | 35 | 18 | 19 |
| HSL | 118 | 115 | 116 |
| p-HSL | 85 | 28 | 68 |

| Diameter (µm) | Control | Protection | Treatment |
|---|---|---|---|
| TNF-α | 18 | 8 | 9 |
| MMP-9 | 18 | 8 | 9 |
| HSL | 9.8 | 9.7 | 9.8 |
| p-HSL | 11.8 | 3.9 | 9.8 |

In the treatment and the protection groups, decreased white oil globulets indicated that lipolytic activity resulted from the activation of p-HSL but not the total from HSL by KMUP-1. HSL partly increased after treatment or protection with KMUP-1 but was also partly changed into p-HSL accompanied by active ATGL. The accompanying changes in the oil globule number and diameter by HSL. Antibody responses, accompanied by a multiple oil globulet number, to p-HSL staining, were more prominent in the protection group than the treatment groups, indicating the major role of p-HSL compared to HSL in inhibiting steatohepatitis by KMUP-1.

Likewise, antibody responses and oil globulet number changes shown by ATGL staining were more prominent in the protection group than the treatment groups, indicating the important role of ATGL in lipolysis induced by KMUP-1 to inhibit steatohepatitis.

### IHC staining of M1 or M2 macrophages in steatohepatitis

F4/80 and CD11c of the classically activated M1 macrophage are the markers in the IHC staining, andCD206 abd CD209a of the M2 macrophage are the markers in the IHC staining. CD209a staining was the most sensitive for the antibody responses. Multiple white oil globulets were found in all HFD-induced liver, indicating hyperadiposity resulting in steatohepatitis. These white oil globulets also interfered with our observation of the color background to separate the contrast difference. The oil globulet number and diameter of M1 type macrophages are as listed in table 5. The KMUP-1 protection group was shown in the right side and the treatment group was shown in the middle of these cytology figures (data not shown). IHC images were obtained under the same light intensities by light microscopy for all specimens. On average, all antibody responses to M1 or M2 type macrophages shared a class 4 saturation of color intensity, as calculated by computer. The oil globulet diameter changes in the protection and the treatment groups were dramatically different from the HFD control.

**Table 5 The amounts and diameter of fat globules in steatohepatitis**

| | Groups | | |
|---|---|---|---|
| Amounts | Control | Protection | Treatment |
| ATGL | 90 | 28 | 46 |
| CD11c-M1 | 43 | 26 | 41 |
| CD209a -M2 | 58 | 41 | 50 |

| Diameter (µm) | Control | Protection | Treatment |
|---|---|---|---|
| ATGL | 18 | 5.5 | 9.8 |
| CD11c-M1 | 16.5 | 8 | 15.5 |
| CD209a -M2 | 13.5 | 9.5 | 11.8 |

**Table 6 The weight change of fat pads in steatohepatitis**

| Weight (mg) | Control | KMUP-1 treatment |
|---|---|---|
| fat pads (L) | 170 | 96 |
| fat pads (R) | 165 | 120 |

### H&E staining of fat pads

HFD-induced hypertrophy/hyperplasia of the white cell type epididymal fat pads, imaged by H&E staining, was inhibited by protection with KMUP-1 (2.5 mg/kg). Table 6 shows that the increased epididymal fat pad weight of mice fed with an HFD was reduced by treatment with KMUP-1 for 14 weeks. KMUP-1 (2.5 mg/kg/day) for 14 weeks significantly reduced the fat pad weight and fat cell diameter during the protection period.

**Table 7 The percentages of diameter and immunoresponse changes**

| Treatment | Control | KMUP-1 |
|---|---|---|
| Fat pads diameter (µm) | 46 | 28 |

| Immunorespons percentages (%) | | |
|---|---|---|
| eNOS | 50.5 | 39 |
| HSL | 34 | 57 |
| IL-10 | 31 | 56 |
| TNF-α | 36 | 22 |

### IHC staining of eNOS/HSL and IL-10/TNF-α in epididymal fat pads

Table 7 shows that HFD for 14 weeks induced IHC staining of eNOS, HSL, IL-10 and TNF-α present on the rim of adipocytes. Protection with KMUP-1 (2.5 mg/kg/day) inhibited the IHC staining of eNOS/TNF-α and increased HSL/IL-10, resulting in the changes of immunoresponse of fat pad cells, respectively.

**Table 8 The percentages of immunoresponse changes of fat pad cells treatment with KMUP-1**

| (%) | Groups | | | | | |
|---|---|---|---|---|---|---|
| | CTL | Protection | KMUP-1HCl treatment (mg/kg) | | | |
| | | | 2.5^{a} | 1 | 2.5 | 5 |
| SR-B1 | 100 | 29 | 59 | | | |
| HMGR | 100 | | | 43 | 150 | 140 |
| ROCK II | 100 | | | 30 | 25 | 10 |
| PPARγ | 100 | | | 53 | 249 | 245 |
| ABCA1 | 100 | | | 98 | 190 | 200 |

| | | | | | | |
|---|---|---|---|---|---|---|
| (note) ^{a}2.5 = 2.5 mg/200 mL drink CTL = Control groups | | | | | | |

**Table 9 The percentages of immunoresponse changes after treatment with Simvastatin**

| (%) | HMGR | ROCKII | PPARγ | ABCA1 |
|---|---|---|---|---|
| CTL | 100 | 100 | 100 | 100 |
| Simva 5mg/kg | 100 | 43 | 280 | 100 |

| | | | | |
|---|---|---|---|---|
| (note) HMGR = HMG CoA reductase CTL= Control groups Simva= Simvastatin, Simvastatinic Acid | | | | |

### HFD-induced HMG CoA reductase, ROCK II, PPARγ and ABCA1 expression in liver

Oral KMUP-1 HCl by gavage affected HMG CoA reductase (HMGR) expression in mice fed with HFD for 8 weeks. Mice fed an HFD showed downregulated HMG CoA reductase expression compared to STD. Both KMUP-1 and DL-ascorbic acid + simvastatin (2.5, 5 mg/kg) and simvastatin (5 mg/kg) significantly reversed HFD-induced downregulation of HMG CoA reductase expression in liver. Additionally, Table 9 showed that KMUP-1 also activated PPARγ and ABCA1 and inactivated ROCK II in animals treated with HFD.

**Table 10 The percentages of HMGR induced by mevalonate**

| HMGR (%) | 10⁻⁹ | 10⁻⁸ | 10⁻⁷ | 10⁻⁶ | 10⁻⁵ | CTL |
|---|---|---|---|---|---|---|
| KMUP-1 | 120 | 125 | 210 | 220 | 270 | 100 |
| Simva | 150 | 152 | 220 | 225 | 280 | 100 |
| (µm) | 20 | 40 | 60 | 80 | 100 | |
| Mevalonate | 110 | 95 | 55 | 48 | 13 | 100 |

| | | | | | | |
|---|---|---|---|---|---|---|
| (note) Simva = Simvastatin, Simvastatinic Acid HMGR = HMG CoA reductase CTL = Control groups Simva vehicle HMGR=120% | | | | | | |

**Table 11 The HMG CoA expression of target drugs in HepG2 cells**

| Expression | | % |
|---|---|---|
| Control group | Vehicle group | 100 |
| Mevalonate (100µM) | | 20 |
| Mevalonate (100µM) +KMUP-1 (10⁻⁵M) | | 62 |
| Mevalonate (100µM) + Simva (10⁻⁵M) | | 50 |

| | | |
|---|---|---|
| (note) Simva = Simvastatin, Simvastatinic Acid | | |

PPARγ is a sensor signaling in lipid metabolism, expressed in adipocytes. ATGL Inhibition of PDE-3B/PDE-5A, leading to activation of HSL, has promised to induce lipolysis of adipocytes by cGMP/PKG. HSL regulates the hydrolysis of acylglycerol and cholesteryl ester in various organs and is activated by PKG to increase the lipolysis of oil droplets.

**Table 12 The expressions of eNOS, PDE-5A, and ROCK II.**

| | eNOS | PDE-5A | ROCK II |
|---|---|---|---|
| Control group | 100 (%) | 100 (%) | 100 (%) |
| KMUP-1 HCl | 155.16±14.8 | 64.5±4.5 | 42.5±6.3 |
| KMUP-1-CMC | 152.14±6.3 | 58.7±3.5 | 38.6±2.7 |
| KMUP-1-VC | 147.21±8.6 | 68.8±5.3 | 40.8±3.7 |

| | | | |
|---|---|---|---|
| (note) KMUP-1-CMC= KMUP-1-sodium carboxyl methylcellulose KMUP-1-VC = KMUP-1-DL-ascorbic acid | | | |

### Serum/vehicle and mevalonate-induced HMG CoA reductase expression

In HepG2 cells supplemented with serum/vehicle-containing medium, expression was concentration-dependently increased by incubation with KMUP-1 or simvastatin (10⁻⁹-10⁻⁵ M) for 24 hours. Application of mevalonate (60, 80, 100 µM) in HepG2 cells concentration-dependently reduced the expression of HMG CoA reductase (Table 10). Mevalonate at 100 µM sharply inhibited HMG CoA reductase expression and this effect was prevented by adding KMUP-1 or simvastatin (10⁻⁵M), indicating the end-product feedback regulation phenomenon of HMG CoA reductase.

### Increased PPARγ/ABCA1/ApoA-I/LXRα

KMUP-1 and simvastatin (10⁻⁹-10⁻⁵ M) increased the expression of PPARγ and ABCA1 in HepG2, suggesting that they could affect the lipid metabolism toward formation of HDL. KMUP-1 increased PPARγ by 2-3 folds which were consistent with the decreased weight gain, indicating that the PPARγ activity of KMUP-1 could be involved in the reduction of body weight (Table 1). Both KMUP-1 and simvastatin (10⁻⁹-10⁻⁵ M) concentration-dependently increased ApoA-I and LXRα expression in HepG2 cells.

**Table 13 The expression of ROCK II, PPARγ and ABCA1**

| | ROCK II | PPARγ | ABCA1 |
|---|---|---|---|
| Control | 100 | 100 | 100 |
| Vehicle | 88 | 260 | 110 |
| Simva (10⁻⁵M) | 45 | 350 | 146 |
| KMUP-1(10⁻⁵ M) | 41 | 595 | 200 |
| C3 exoenzyme (5 ng/ml) | 32 | 500 | 210 |
| Y27632 (10⁻⁵M) | 52 | 550 | 225 |
| Rp-8-pCPT-cGMPS (10 µM) | 320 | | |
| Rp-8-pCPT-cGMPS +KMUP-1 (10 µM) | 170 | | |

| | | | |
|---|---|---|---|
| **cGMP-pathway and RhoA/ROCK II** | | | |

Both RhoA antagonist C3 exoenzyme (5 ng/ml) and ROCK antagonist Y27632 (10 µM) reduced ROCK II expression, which was increased by 10 µM cGMP antagonist Rp-8-pCPT-cGMPS (8-(p-chloro-phenylthio)guanosine-3,5-monophosphorothioate) and inhibited by the combination with KMUP-1 (10 µM), indicating the involvement of a cGMP-pathway in HepG2 cells. In addition, KMUP-1, simvastatin, C3 exoenzyme and Y27632 all increased PPARγ and ABCA1 expression. KMUP-1 also increased PPARγ in parallel with the decreased weight gain, indicating that PPARγ has an important role in decreasing weight gain.

**Table 14 RhoA/ROCK II activation in the presence of GGPP and FPP**

| (10 µM) | GGPP | | FPP | |
|---|---|---|---|---|
| | ROCKII | PPARγ | ROCKII | PPARγ |
| % | 150 | 50 | 118 | 64 |
| Control | 100 | 100 | 100 | 100 |
| KMUP-1 10⁻⁹ | 112 | 78 | 84 | 72 |
| 10⁻⁸ | 120 | 90 | 86 | 84 |
| 10⁻⁷ | 108 | 108 | 88 | 142 |
| 10⁻⁶ | 60 | 106 | 80 | 140 |
| 10⁻⁵ | 40 | 120 | 60 | 138 |

| | | | | |
|---|---|---|---|---|
| (note) GGPP= Geranylgeranyl pyrophosphate FPP = Farnesyl pyrophosphate | | | | |

**Table 15 ROCK II and PPARγ activation in the presence of GGPP**

| (10 µM) | GGPP | |
|---|---|---|
| | ROCKII | PPARγ |
| % | 220 | 50 |
| Control | 100 | 100 |
| Simva 10⁻⁹ | 210 | 46 |
| 10⁻⁸ | 214 | 40 |
| 10⁻⁷ | 200 | 44 |
| 10⁻⁶ | 220 | 46 |
| 10⁻⁵ | 210 | 46 |

| | | |
|---|---|---|
| (note) Simva = Simvastatin, Simvastatinic Acid | | |

Application of exogenous GGPP and FPP increased RhoA/ROCK II expression, and KMUP-1 (10⁻⁹-10⁻⁵ M) attenuated this phenomenon in HepG2 cells. In contrast, simvastatin did not make ROCK II inactivated in the presence of exogenous GGPP and FPP.

### Exogenous GGPP or FPP decreases PPARγ and ABCA1

Incubation of HepG2 cells with FPP or GGPP (10 µM) alone suppressed the expression of PPARγ and ABCA1. Incubation of FPP or GGPP with KMUP-1 (10⁻⁹-10⁻⁵ M) reversed the expression of PPARγ and ABCA1, but simvastatin did not affect PPARγ in the presence of GGPP (10 µM).

### Biosynthesis of [¹⁴C]mevalonate

KMUP-1 (10 µM) could not reduce [¹⁴C]mevalonate formation. In contrast, simvastatin inhibited [¹⁴C]mevalonate formation by 86.6±4.2%, compared to the vehicle group. [¹⁴C]HMG CoA reductase of >90% in purity and 20 units/mg in the activity form (Sigma-Aldrich, St. Louis, MO, U.S.A.) was used to determine [¹⁴C]mevalonate formation.

### IHC of LDLRs and expression of PKG/PKA

HFD-induced LDLRs expression in the liver was estimated using IHC staining methods. Notably, KMUP-1 HCl increased the hepatic LDLRs of animals fed with HFD in both the protection and treatment groups. Western blotting of LDLRs and PKA/PKG showed that KMUP-1 (10, 20, 40 µM) could not significantly affect PKA protein expression in HepG2 cells in the presence of LDL (500 µg/mL), a pathologic model of hyperlipidemia, but increased the expression of PKG. However, KMUP-1 (1, 10, 100 µM) reversed the 200 µg/mL oxidized low density lipoprotein, oxidized LDL (oxLDL)-induced reduction of PKA expression.

### Fluoroscent staining of cellular LDLRs/PKA/HSL

HepG2 cells were stained with fluorescence and treated with different concentrations of KMUP-1 or simvastatin for 24 hours. The results showed increased LDLR (green fluorescence) expression in HepG2 cells with different concentrations of KMUP-1 (10⁻⁶, 10⁻⁵, 10⁻⁴ M) or simvastatin (10⁻⁵ M). The expression intensity of HepG2 cells treated with KMUP-1 was compared to the control. KMUP-1 at concentrations >10⁻⁴ M, for unknown reasons, showed a decline in fluoresence, suggesting that such a concentration could be near the viability range of HepG2 cells. PKA and HSL (green fluorescence) also showed increased immunoreactivities in HepG2 cells treated with KMUP-1 or simvastatin. However, PKG immunoreactivity was not significantly affected by KMUP-1 (data not shown).

A pharmaceutical composition is disclosed in the present application, in which the different active agents being consisting of a PDE-5 inhibitor and a Statin analogue for treating and/or preventing lipid metabolism disorder. Oral PDE-5 inhibitors and Statin analogue were administered together in C57BL/6J male mice which were fed a high-fat-diet by gavage for 8 weeks. The targeted drugs inhibited HFD-induced plasma and liver TG, total cholesterol and LDL, increased HDL/HMGR/ROCK II/PPARγ/ABCA1 expression and increased SR-B 1, PKA/PKG expression and LDL receptors (LDLRs)/PKA immunoreactivity.

### Materials and Methods

### Animals and serum

In the experiment, C57BL/6J male mice (21-22 g), after fasting, were fed an HFD as a model of hyperlipidemia for 8 weeks. During the 2 month experiments, mice fasted for one night before the experiment was changed from a standard diet (STD) to an HFD and then the mice were randomly divided into 13 groups, including a protection group, 2 control and 10 treatment groups. Six mice were used in each group. The control mice received either STD or HFD during 14 weeks and the treatment groups mice were fed HFD and treated with 2.5 and 5 mg/kg/day of KMUP-1 HCl, Sildenafil, simvastatin, KMUP-1 HCl + simvastatin, KMUP-1-DL-ascorbic acid + simvastatin, Sildenafil + simvastatin, Tadalafil+simvastatin, or other target drugs administered by gavage during the last 6 weeks for the purpose of analysis experiments. Liver from the protection group supplemented with KMUP-1 HCl (1 mg/kg/day) were used to determine the HMG CoA reductase expression. Each group of mice had free access to drinking water from week 1 to week 14. Tap water was used to normalize mineral nutrition.

Animals were housed in the animal center with a day-night cycle system at Kaohsiung Medical University, Kaohsiung, Taiwan. All procedures and protocols were approved by the Animal Care and Use Committee at Kaohsiung Medical University and complied with the *Guide for the Care and Use of Laboratory Animals* published by the U.S. National Institutes of Health.

### Biochemical analysis of serum

Within 2 months, the 3 day food intake average for each animal was measured. The weight gain and plasma lipid levels of each group were determined and compared to the non-treatment control group. Mouse blood was collected during the daytime by cardiac puncture followed by centrifugation at 90× g (Benchtop Centrifuge, U.S.A.) to separate the serum, and frozen at -80°C, for biochemical analysis using a Hitachi Clinical Analyzer 7070 (Hitachi High-Technologies Co. Tokyo, Japan). Agents used in the assays were obtained from Merck & Co. (Kenilworth, NJ, U.S.A.). Triglyceride (TG), total cholesterol, HDL cholesterol and LDL cholesterol in mouse serum were measured by methods used in the clinic. To measure the hepatic Triglyceride, isolated liver were cut into small chips.

### Cell culture

The HepG2 hepatoma cell line was purchased from the American Type Culture Collection (ATCC, Manassas, VA, U.S.A.). Cells were cultured in Dulbecco's Modified Eagle's Medium (DMEM). The culture media was supplemented with 5% heat-inactivated FBS, penicillin (100 U/mL) and streptomycin (100 µg/mL). Cells were grown in a humidified atmosphere containing 5% CO₂ at 37°C, in which the oxygen tension in the incubator was held at 140 mmHg (20% O₂, v/v; normoxic conditions). With respect to the solubility difference between the targeted drugs, suitable solvents were considered and selected, and, therefore, KMUP-1 HCl was dissolved in distilled water and simvastatin in a vehicle (propylene glycol). Then the targeted drugs solution was incubated with the cells for 24 hours, followed by protein extraction. The targeted drugs were added to the final concentration vehicle- or distilled water-treated cells, to observe the changes of propylene glycol HMG CoA reductase expression in the medium and never exceeded 0.1% HepG2 cells.

### Western blotting analysis of protein expression in HepG2 cells and livers

HepG2 cells were treated with various concentrations of targeted drugs for 24 hours. Reactions were terminated by washing twice with cold PBS, and the cells were then harvested. Proteins in the whole-cell lysate were homogenized in an ice-cold lysis buffer and a protease inhibitor (Sigma-Aldrich, St. Louis, MO, U.S.A.). The homogenate was centrifuged at 90,000× g for 15 minutes at 4°C, and supernatant was recovered as the total cellular protein. Cytosolic and membrane fractions of HepG2 cells were prepared using a CNM (Cytosol Nuclear Membrane) compartmental protein extraction kit (BioChain Institute Inc., Hayward, CA, U.S.A.) according to the manufacturer's instructions. All of the fractionated protein solutions were stored at -80°C until analysis. To measure the expression levels of proteins by drugs, the total cell protein was extracted after incubation with treatments for 24 hours and then Western blotting analysis was performed as described previously.

### The expression of SR-B1, HMGR, PPARγ and ROCK II

For the expression of SR-B1, HMGR, PPARγ and ROCK II, isolated liver tissues were cut into small chips and placed into an extraction buffer (Tris 10 mM, pH 7.0, NaCl 140 mM, PMSF 2 mM, DTT 5 mM, NP-40 0.5%, pepstatin A 0.05 mM and leupeptin 0.2 mM) for hepatic protein extraction, and centrifuged at 20,000× g for 30 minutes. The obtained protein extract was boiled to a ratio of 4:1 with a sample buffer (Tris 100 mM, pH 6.8, glycerol 20%, SDS 4% and bromophenol blue 0.2%). Electrophoresis was performed using 10% SDS-polyacrylamide gel (1 hour, 100 V, 40 mA, 20 µg protein per lane).

Separated proteins, after 3 repeated centrifugations to discard up-layer tissue lipid inpurity, were transferred to PVDF membranes treated with 5% fat-free milk powder to block the nonspecific IgGs (90 minutes, 100 V) and incubated for 1 hour with specific protein antibody. The blot was then incubated with anti-mouse or anti-goat IgG linked to alkaline phosphatase (1:1000) for 1 hour.

### HMG CoA reductase activity and [¹⁴C]mevalonate formation

Human recombinant HMG CoA reductase expressed in *E. coli* (H7039, Sigma-Aldrich, MO, U.S.A.) was used. Human recombinant HMG-CoA reductase, examined by SDS-PAGE to be ≥90% in purity, 2-8 units/mg protein in activity and ∼76kDa in molecular weight (H7039, Sigma-Aldrich, St. Louis, MO. U.S.A.), was used for determining the formation of [¹⁴C]mevalonate. KMUP-1 and targeted drugs were pre-incubated with 35 ng/ml enzyme in phosphate buffer pH 7.5 for 15 minutes at 37°C. The reaction was initiated by the addition of 2.5 µM [¹⁴C]HMG-CoA for another 20 minutes incubation period and terminated by further addition of 1 N HCl. An aliquot was removed by column and counted to determine the amount of [¹⁴C]mevalonate formed (Ricera^{®} Co. Ltd., Taipei, Taiwan).

### cGMP pathway and RhoA/ROCK II

To confirm that RhoA antagonist C3 exoenzyme (5 µg/ml) and ROCK antagonist Y27632 (10 µM), dissolved in 10% popyleneglycol, can inactivate the expression of ROCK II, they were added to cells in culture for 24 hours, respectively, to measure the expression of ROCK II and related expressions of PPARγ and ABCA-1 in HepG2 cells.

To confirm that the cGMP antagonist Rp-8-pCPT-cGMPs (10 µM), dissolved in 10% propylene glycol can increase the expression of ROCK II and that KMUP-1 or targeted drugs can reduce Rp-8-pCPT-cGMPs-induced expression of ROCK II, HepG2 cells were pre-incubated with Rp-8-pCPT-cGMPs for 30 minutes as a control and then in combination with KMUP-1 (10 µM) or targeted drugs for 24 hours.

### Immunohistochemistry (IHC) staining of LDLRs in livers

Liver tissues were fixed in 10% buffered formalin for 24 hours and then embedded in paraffin. The paraffin-embedded liver tissue sections (4 µm thick) were first heat immobilized and deparaffinized using xylene and then rehydrated in a graded series of ethanol, followed by a final wash in distilled water. Finally, tissue sections were stained with Periodic Acid-Schiff (PAS) and Mayer's hematoxylin solution.

### Expression and fluorescence staining of LDLRs in the presence of exogenous LDL

HepG2 cells were used to determine the cellular protein expression of LDLRs in the presence of exogenous LDL (500 µg/mL). Bodipy-493/503 (green) and LDLRs on HepG2 cells were detected with a secondary anti-body conjugated to Cy3 (red) overnight at 4°C, followed by merger of obtained BODIPY-and LDL-images to analyze the location of LDLRs. All images were collected and analyzed by scanning with Nikon Eclipse TE200-S microscope (Tokyo, Japan).

### Expression of PKA/PKG and immunoreactivity of PKA/HSL

To determine that KMUP-1 or targeted drugs can affect PKA, we incubated KMUP-1 (10⁻⁴, 10⁻⁵, 10⁻⁶ M) or targeted drugs with HepG2 cells for 24 hours to measure the protein expressions of PKA/PKG by Western blotting or PKA/PKG and HSL immunoreactivities by fluorescence staining, combined with image scanning in the absence or presence of oxidized LDL (200 µg/mL).

### Measurement of hepatic oil globulet diameter

The diameter of the white bar in the photograph was standardized at 100 µM for measuring the specific diameter of oil globulets in a whole liver slice, observed by microscope, with the aid of Powerpoint PC computer software (Microsoft^{®}, U.S.A.) and printed out by a Hewlett-Packard^{®} printer (U.S.A.). An increase in oil globulet dimeter and cell number indicated increasing liver steatosis, i.e. steaotohepatitis. In contrast, decrease in diameter and the number of fat cells indicated the inhibition of steaotohepatitis. Oil globulets on liver slices were observed with a Nikon Eclipse TE200-S microscope. Hematoxylin-eosin (H&E) and IHC staining of liver were used to assess oil globulet numbers under a microscope.

### Hematoxylin-Eosin (H&E) staining of liver and epididymal fat pads

Mice liver and epididymal fat pads were cut and soaked in formalin, dehydrated through graded alcohols and embedded in paraffin. Specimens of liver tissues and epididymal fat pads fixed with formalin (4%) were embedded in paraffin for one hour at 4°C, cut into 4-µm-thick sections, and subjected to H&E staining before examination by light microscope.

### IHC staining of MMP-9/TNFα, HSL/p-HSL/ATGL and eNOS/IL-10)

For IHC of MMP-9/TNFα, HSL/p-HSL/ATGL and eNOS/IL-10, antigen retrieval of de-paraffinated sections was performed in Dako target retrieval solution, pH 9.0, in a vegetable steamer followed by the quenching of endogenous peroxidase activity with 3.0% H₂O₂ in methanol. Sections were then incubated with specific primary antibodies overnight at 4°C in a humidified chamber. The antibodies used included rabbit polyclonal anti-eNOS (1:50 dilution) (Abcam, Cambridge, UK) or rabbit monoclonal anti-cleaved caspase-3 (1:50 dilution) (Cell Signaling, U.S.A.). The sections were then examined using a DAKO REAL EnVision^{™} Detection System kit (DAKO, Carpinteria, CA, U.S.A.) and counterstained with hematoxylin. Images were obtained through a Nikon Eclipse TE200-S microscope.

### Materials and reagents

Immunoreactive bands were visualized using horseradish peroxidase-conjugated secondary antibodies and subsequent ECL detection (GE Healthcare Bio-Sciences Corp., Piscataway, NJ, U.S.A.). Mouse or rabbit monoclonal antibody for ROCK II (Upstate, Lake Placid, NY, U.S.A.), RhoA (Santa Cruz Biotechnology, Santa Cruz, CA, U.S.A.), HMG CoA reductase (Upstate, Lake Placid, NY, U.S.A.), PPARγ (Abcam, Cambridge, UK), ABCA-1 (Cell Signaling, Boston, MA, U.S.A.), ApoA-I (Abcam, Cambridge, UK), LXRα (Santa Cruz Biotechnology, Santa Cruz, CA, U.S.A.), LDLR (Abcam, Cambridge, UK), HSL (Cell Signaling, Boston, MA, U.S.A.), eNOS (Abcam, Cambridge, UK), MMP-9 (Abcam, Cambridge, UK), TNFα (Abcam, Cambridge, UK), IL-10 (Abcam, Cambridge, UK) and the loading control protein β-actin (Sigma-Aldrich, St. Louis, MO, U.S.A.) were used in our Western blot analyses. Rabbit polyclonal antibody was used to recognize both PPARγ1 and PPARγ2 in the experiments. Rp-8-pCPT-cGMPs and C3 exoenzyme were purchased from Sigma-Aldrich (St. Louis, MO, U.S.A.). HFD was a basal purified diet (W/60% energy from fat, Blue:58G9 Test Diet; Richmond, VA, U.S.A.). LDL was purchased from Abcam (Cambridge, UK). Oxidized LDL (oxLDL) was purchased from Biomedical Technologies Inc. (Stoughton, MA, U.S.A.). Human recombinant HMG-CoA reductase (H7039, Sigma-Aldrich, St. Louis, MO. U.S.A.), examined by SDS-PAGE to be ≥90% in purity, 2-8 units/mg protein in activity and ∼76kDa in molecular weight was used to determine the formation of [¹⁴C]mevalonate.

### Statistical evaluation

The experimental results were expressed as means ± SE. Statistical differences were determined by independent and paired Student's *t*-test in unpaired and paired samples, respectively. Whenever a control group was compared with more than one treated group, one way ANOVA or two way repeated measures ANOVA was used. When the ANOVA showed a statistical difference, the Dunnett's or Student-Newman-Keuls test was applied. A *P* value less than 0.05 was considered significant in all experiments. Analysis of the data and plotting of the figures were done using SigmaPlot software (Version 8.0, Chicago, IL, U.S.A.) and SigmaStat (Version 2.03, Chicago, IL, U.S.A.) run on an IBM compatible computer.

### Example 1: Combination drug for the treatment of the lipid metabolism disorder

| | |
|---|---|
| KMUP-1 HCl | (8.6 g) |
| Simvastatin sodium salt | (4.6 g) |

### Example 2: Combination drug for the treatment of the lipid metabolism disorder

| | |
|---|---|
| KMUP-1 HCl | (8.6 g) |
| Pravastatin sodium salt | (4.5 g) |

### Example 3: Combination drug for the treatment of the lipid metabolism disorder

| | |
|---|---|
| KMUP-1 HCl | (8.6 g) |
| Simvastatin sodium salt | (4.6 g) |

### Example 4: Combination drug for the treatment of the lipid metabolism disorder

| | |
|---|---|
| KMUP-1 DL-ascorbic acid complex | (11.5 g) |
| Simvastatin sodium salt | (4.6 g) |

### Example 5: Preparation of the tablets

Tablets were prepared using standard mixing and formulation techniques as described in U.S. Pat. No. 5,358,941, to Bechard et al., issued Oct. 25, 1994, which is incorporated by reference herein in its entirety.

| | |
|---|---|
| KMUP-2 DL-ascorbic acid complex | 100 mg |
| Simvastatin sodium salt | 100 mg |
| Lactose | qs |
| Corn starch | qs |

### Example 6: Combination drug for the treatment of the lipid metabolism disorder

| | |
|---|---|
| Sildenafil citratel complex | (13.2 g) |
| Pravastatin sodium salt | (4.5 g) |

### Example 7: Combination drug for the treatment of the lipid metabolism disorder

| | |
|---|---|
| Avanafil | (9.6 g) |
| Pravastatin sodium salt | (4.5 g) |

### Example 8: Combination drug for the treatment of the lipid metabolism disorder

| | |
|---|---|
| Tadalafil | (7.7 g) |
| Simvastatin sodium salt | (4.6 g) |

### Example 9: Combination drug for the treatment of the lipid metabolism disorder

| | |
|---|---|
| KMUP-1 oleic acid complex | (13.7 g) |
| Pravastatin sodium salt | (4.5 g) |

### Example 10: Preparation of the tablets

Tablets were prepared using standard mixing and formulation techniques as described in U.S. Pat. No. 5,358,941, to Bechard et al., issued Oct. 25, 1994, which is incorporated by reference herein in its entirety.

| | |
|---|---|
| Avanafil | 100 mg |
| Pravastatin sodium salt | 100 mg |
| Lactose | qs |
| Corn starch | qs |

### Example 11: Combination drug for the treatment of the lipid metabolism disorder

| | |
|---|---|
| KMUP-1 oleic acid complex | (13.7 g) |
| Fluvastatin sodium salt | (4.4 g) |

### Example 12: Combination drug for the treatment of the lipid metabolism disorder

| | |
|---|---|
| Dipyridamole | (9.9 g) |
| Fluvastatin sodium salt | (4.4 g) |

### Example 13: Combination drug for the treatment of the lipid metabolism disorder

| | |
|---|---|
| KMUP-1 DL-ascorbic acid complex | (11.5 g) |
| Fluvastatin sodium salt | (4.4 g) |

### Other Embodiments

1. A method for inhibiting a lipid metabolism disorder, including steps of: providing a subject suffering from the lipid metabolism disorder; and administering a therapeutically effective amount of compound being one selected from the group consisting of KMUPs derivative, KMUPs derivative complex (KMUPs-RX), Sildenafil analogue and Sildenafil analogue complex (Sildenafil analogue-RX) and a Statin analogue to the subject in need thereof.
2. The method of Embodiment 1, wherein the Statin analogue is one selected from the group consisting of Atorvastatin, Cerivastatin, Fluvastatin, Lovastatin, Mevastatin, Pravastatin, Rosuvastatin, Simvastatin, Pravastatin acid, and their pharmaceutically acceptable salts.
3. The method of Embodiment 1 or 2, wherein the KMUPs derivative is one selected from the group consisting of KMUP-1, KMUP-2, KMUP-3 and KMUP-4, and their pharmaceutically acceptable salts.
4. The method of any one of Embodiments 1-3, wherein the Sildenafil analogue is one selected from the group consisting of Acetidenafil, Cinnamyldenafil, Dimethyl-Acetidenafil, Dioxy-Acetidenafil, Hydroxy-Acetidenafil, Nor-Acetidenafil, Oxyhongdenafil, Gendenafil, Carbodenafil, Acetil Acid, Chlorodenafil, Piperildino-Acetidenafil, Isopiperazinonafil, Piperazinonafil, Nitrodenafil, Benzyl-Sildenafil, Cyclopenylnafil, N-desmethyl-Sildenafil, Hydroxyhomo-Sildenafil, Homo-Sildenafil, Propoxyphentyl-Sildenafil, Propoxyphentyl-hydroxyhomoSildenafil, Sildenafil, Aildenafil, Propoxyphentyl-Aildenafil, Normeo-Sildenafil, Descarbon-Sildenafil, Udenafil, Gisadenafil, Mirodenafil, Vardenafil, ThioAildenafil, Propoxy phentylthioAildenafil, ThioSildenafil, Sulfohomosildenafil, Hydroxythiohomosildenafil and Avanafil, and their pharmaceutically acceptable salts.
5. The method of any one of Embodiment 1-4, wherein the warm-blooded animal is one selected from the group consisting of a human, a goat, a lamb, a sheep, a pig, a cow, a chicken, and a duck.
6. The method of any one of Embodiments 1-5, including a step of: combined administering a pharmaceutically effective amount of compound to a warm-blooded animal suffering from the lipid metabolism disorder in need thereof.
7. The method of any one of Embodiments 1-6, wherein the lipid metabolism disorder is one selected from the group consisting of non-alcoholic fatty liver disease, hyperadiposity, dyslipidemia, hepatic steaotosis, high-fat-diet-induced obesity, insulin resistance, high-fat-diet-induced lipid accumulation combined with a symptom of one of inflammation and liver damage, and any combination thereof.
8. The method of any one of Embodiments 1-7, wherein the administration is performed by one selected from the group consisting of an oral, an injection, an inhalation and a topical administration.
9. A method for inhibiting a lipid metabolism disorder, including steps of: providing a subject suffering from the disease; and administering a pharmaceutically effective amount of compound being one selected from the group consisting of PDE-5 inhibitors of the first type and second type, and administering a pharmaceutically effective amount of a Statin analogue to the subject in need thereof in a combined dosage between 1 and 5.0 milligrams per kilogram of the body weight.
10. The method of Embodiment 9, wherein the first type PDE-5 inhibitor is one selected from the group consisting of KMUPs derivative, KMUPs derivative complex, Sildenafil analogue and Sildenafil analogue complex.
11. The method of Embodiment 9 or 10, wherein the Sildenafil analogue includes Sildenafil, Hydroxyhomosildenafil, Desmethylsildenafil, Acetidenafil, Udenafil, Vardenafil and Homosildenafil, and their pharmaceutically acceptable salts.
12. The method of any one of Embodiments 9-11, wherein the KMUPs derivative is one selected from the group consisting of KMUP-1, KMUP-2, KMUP-3 and KMUP-4, and their pharmaceutically acceptable salts.
13. The method of any one of Embodiments 9-12, wherein the Statin analogue is one selected from the group consisting of Atorvastatin, Cerivastatin, Fluvastatin, Lovastatin, Mevastatin, Pravastatin, Rosuvastatin, Simvastatin, Pravastatin acid, and their pharmaceutically acceptable salts.
14. The method of any one of Embodiments 9-13, wherein the second type PDE5 inhibitor is one selected from the group consisting of Avanafil, Benzamidenafil, Dasantafil, Dipyridamole, E4021, Icariin, Rolipram, Piclamilast, Tadalafil and Zaprinast, and their pharmaceutically acceptable salts.
15. The method of any one of Embodiments 9-14, wherein the pharmaceutical composition has the beneficial effects of removing plasma LDL via increasing LDLRs, increasing circulation and hepatic fat loss via HSL which is around the lipid droplets of adipocytes in the entire body and the sites of lipid storage in hepatic cells.
16. The method of any one of Embodiments 9-15, wherein the lipid metabolism disorder results in decrease in the body weight or adipose tissue, increase in fat oxidation or insulin sensitivity, and/or decrease of the inflammation or oxidative stress, and includes one selected from the group consisting of non-alcoholic fatty liver disease, hyperadiposity, dyslipidemia, hepatic steaotosis, high-fat-diet-induced obesity, insulin resistance, high-fat-diet-induced accumulation with a symptom of one of inflammation and liver damage, and any combination thereof.
17. The method of any one of Embodiments 9-16, wherein the warm-blooded animal is one selected from the group consisting of a man, a goat, a lamb, a pig, a cow, a chicken, and a duck.
18. The method of any one of Embodiments 9-17,wherein the lipid metabolism disorder is one selected from the group consisting of non-alcoholic fatty liver disease, hyperadiposity, dyslipidemia, hepatic steaotosis, high-fat-diet-induced obesity, insulin resistance, high-fat-diet-induced lipid accumulation combined with a symptom of inflammation and liver damage, and any combination thereof.
19. The method of any one of Embodiments 9-18, wherein the administration is performed by one selected from an oral, an injection, an inhalation and a topical administration.
20. A method for inhibiting a lipid metabolism disorder, including steps of: providing a subject suffering from the lipid metabolism disorder; and administering a pharmaceutical composition including one selected from the group consisting of a KMUPs derivative, KMUPs derivative complex (KMUPs-RX), Sildenafil analogue and a Sildenafil analogue complex (Sildenafil analogue-RX) compound, and a Statin analogue to the subject at a dosage between 1 and 5.0 milligrams per kilogram of the body weight, wherein KMUPs derivative includes KMUP-1, KMUP-2, KMUP-3 and KMUP-4; RX includes a carboxylic group selected from the group consisting of D-ascorbic acid, L-ascorbic acid, DL-ascorbic acid, oleic acid, phosphoric acid, citric acid, nicotinic acid, sodium carboxymethylcellulose (sodium CMC), hyaluronic acid, polyacrylic acid (PAA), polymethylmethacrylates (PMMA), Eudragit, dextran sulfate, heparan sulfate, polylactic acid or polylactide (PLA), polylactic acid sodium (PLA sodium), polyglycolic acid sodium (PGCA sodium), poly-γ-polyglutamic acid sodium (γ-PGA sodium), γ-polyglutamic acid (γ-PGA), alginate-poly-1-lysine-alginate (APA) and poly-γ-polyglutamic acid derivative.
21. The method of any one of Embodiments 9-20, wherein the Sildenafil analogue is one selected from the group consisting of Acetidenafil, Avanafil, DesmethylSildenafil, Gisadenafil, HomoSildenafil, HydroxyhomoSildenafil, Mirodenafil, Lodenafil Carbonate, Sildenafil, Udenafil, Vardenafil, and their pharmaceutically acceptable salts.

### References:

1. Dai Z.-K., et al., (2014) Xanthine Derivative KMUP-1 Reduces Inflammation and Hyperalgesia in a Bilateral Chronic Constriction Injury Model by Suppressing MAPK and NFκB Activation. Mol. Pharm., 11, 1621-1631.
2. Bivalacqua T. J., et al., (2013) Sildenafil citrate-restored eNOS and PDE5 regulation in sickle cell mouse penis prevents Priapism via control of oxidative/nitrosative stress. PLoSONE, 8: e68028.
3. Chung H.-H., et al., (2010) The xanthine derivative KMUP-1 inhibits models of pulmonary artery hypertension via increased NO and cGMP-dependent inhibition of RhoA/Rho kinase. Br. J. Pharmacol. 160: 971-986.
4. Chung H.-H., et al., (2010) KMUP-1 inhibits pulmonary artery proliferation by targeting serotonin receptors/transporter and NO synthase, inactivating RhoA and suppressing AKT/ERK phosphorylation. Vascu. Pharmacol. 53: 239-249.
5. Kuo, K.-K., et al., (2015) Xanthine-based KMUP-1 Improves HDL via PPARγ/SR-B1, LDL via LDLRs, and HSL via PKA/PKG for Hepatic Fat Loss. J. Lipid Res. 56(11): 2070-2084.
6. Das, A., et al., (2015) PDE5 Inhibitors as Therapeutics for Heart Disease, Diabetes and Cancer. Pharmacol. Ther. 147: 12-21.
7. Zhuge F., et al., (2016) DPP-4 Inhibition by Linagliptin Attenuates Obesity-related Inflammation and Insulin Resistance by Regulating M1/M2 Macrophage Polarization. Diabetes, 65(10): 2966-2979.
8. Meshkani R., et al., (2016) Tissue Resident Macrophages: Key Players in the Pathogenesis of Type 2 Diabetes and its Complications, Clin. Chim. Acta, 26: 462:77-89.

## Claims

1. A method for inhibiting a lipid metabolism disorder, **characterized by** comprising:
administering a therapeutically effective amount of compound being one selected from the group consisting of phosphodiesterase 5 (PDE-5) inhibitors of the first type and second type and a Statin analogue to a warm-blooded animal suffering from the lipid metabolism disorder.

2. The method as claimed in Claim 1, **characterized in that** the first type PDE-5 inhibitor is one selected from the group consisting of KMUPs derivative, KMUPs derivative complex (KMUPs-RX), Sildenafil analogue and Sildenafil analogue complex (Sildenafil analogue-RX), and RX includes a carboxylic group selected from the group consisting of D-ascorbic acid, L-ascorbic acid, DL-ascorbic acid, oleic acid, phosphoric acid, citric acid, nicotinic acid, sodium carboxymethylcellulose (sodium CMC), hyaluronic acid, polyacrylic acid (PAA), polymethacrylates (PMMA), Eudragit, dextran sulfate, heparan sulfate, polylactic acid or polylactide (PLA), polylactic acid sodium (PLA sodium), polyglycolic acid sodium (PGCA sodium), poly-γ-polyglutamic acid sodium (γ-PGA sodium), γ-polyglutamic acid (γ-PGA), alginate-poly-1-lysine-alginate (APA) and poly-γ-polyglutamic acid derivative.

3. The method as claimed in Claim 1 or 2, **characterized in that** the KMUPs derivative is one selected from the group consisting of KMUP-1, KMUP-2, KMUP-3 and KMUP-4, and their pharmaceutically acceptable salts.

4. The method as claimed in any of Claims 1-3, **characterized in that** the Sildenafil analogue is one selected from the group consisting of Acetidenafil, Cinnamyldenafil, Dimethyl-Acetidenafil, Dioxy-Acetidenafil, Hydroxy-Acetidenafil, Nor-Acetidenafil, Oxyhongdenafil, Gendenafil, Carbodenafil, Acetil Acid, Chlorodenafil, Piperildino-Acetidenafil, Isopiperazinonafil, Piperazinonafil, Nitrodenafil, Benzyl-Sildenafil, Cyclopenylnafil, N-desmethyl-Sildenafil, Hydroxyhomo-Sildenafil, Homo-Sildenafil, Propoxyphentyl-Sildenafil, Propoxyphentyl-hydroxyhomoSildenafil, Sildenafil, Aildenafil, Propoxyphentyl-Aildenafil, Normeo-Sildenafil, Descarbon-Sildenafil, Udenafil, Gisadenafil, Mirodenafil, Vardenafil, ThioAildenafil, Propoxy phentylthioAildenafil, ThioSildenafil, Sulfohomosildenafil, Hydroxythiohomosildenafil and Avanafil, and their pharmaceutically acceptable salts.

5. The method as claimed in any of Claims 1-4, **characterized in that** the second type PDE-5 inhibitor is one selected from the group consisting of Avanafil, Benzamidenafil, Dasantafil, Dipyridamole, E4021, Icariin, Rolipram, Piclamilast, Tadalafil and Zaprinast, and their pharmaceutically acceptable salts.

6. The method as claimed in any of Claims 1-5, **characterized in that** the Statin analogue is one selected from the group consisting of Atorvastatin, Cerivastatin, Fluvastatin, Lovastatin, Mevastatin, Pravastatin, Rosuvastatin, Simvastatin, Pravastatin acid, and their pharmaceutically acceptable salts.

7. The method as claimed in any of Claims 1-6, **characterized in that** the warm-blooded animal is one selected from the group consisting of a human, a goat, a sheep, a pig, a cow, a chicken, and a duck.

8. The method as claimed in any of Claims 1-7, **characterized in that** the lipid metabolism disorder is one selected from the group consisting of liver disease, non-alcoholic fatty liver disease, hyperadiposity, dyslipidemia, hepatic steaotosis, high-fat-diet-induced lipid accumulation, high-fat-diet-induced obesity, insulin resistance, high-fat-diet-induced lipid accumulation combined with a symptom of one of inflammation and liver damage, and any combination thereof.

9. A pharmaceutical composition for inhibiting a lipid metabolism disorder, **characterized by** comprising:
a therapeutically effective amount of compound being one selected from the group consisting of phosphodiesterase 5 (PDE-5) inhibitors of the first type and second type and a Statin analogue and configured to be administered to a warm-blooded animal suffering from the lipid metabolism disorder.

10. The pharmaceutical composition as claimed in Claim 9, **characterized in that** the pharmaceutical composition acts as a peroxisome proliferator activated receptor (PPAR) agonist, lowers plasma low-density of lipoprotein (LDL) by increasing low density lipoprotein receptor (LDLRs) function, and facilitates fat loss by facilitating hormone-sensitive lipase (HSL) function.

11. The pharmaceutical composition as claimed in Claim 9 or 10, **characterized in that** the first type PDE-5 inhibitor is one selected from the group consisting of KMUPs derivative, KMUPs derivative complex, Sildenafil analogue and Sildenafil analogue complex.

12. The pharmaceutical composition as claimed in any of Claims 9-11, **characterized in that** the KMUPs derivative is one selected from the group consisting of KMUP-1, KMUP-2, KMUP-3 and KMUP-4, and their pharmaceutically acceptable salts, and the Sildenafil analogue is one selected from the group consisting of Acetidenafil, Cinnamyldenafil, Dimethyl-Acetidenafil, Dioxy-Acetidenafil, Hydroxy-Acetidenafil, Nor-Acetidenafil, Oxyhongdenafil, Gendenafil, Carbodenafil, Acetil Acid, Chlorodenafil, Piperildino-Acetidenafil, Isopiperazinonafil, Piperazinonafil, Nitrodenafil, Benzyl-Sildenafil, Cyclopenylnafil, N-desmethyl-Sildenafil, Hydroxyhomo-Sildenafil, Homo-Sildenafil, Propoxyphentyl-Sildenafil, Propoxyphentyl- hydroxyhomoSildenafil, Sildenafil, Aildenafil, Propoxyphentyl-Aildenafil, Normeo-Sildenafil, Descarbon-Sildenafil, Udenafil, Gisadenafil, Mirodenafil, Vardenafil, ThioAildenafil, Propoxy phentylthioAildenafil, ThioSildenafil, Sulfohomosildenafil, Hydroxythiohomosildenafil and Avanafil, and their pharmaceutically acceptable salts.

13. The pharmaceutical composition as claimed in any of Claims 9-12, **characterized in that** the second type PDE-5 inhibitor is one selected from the group consisting of Avanafil, Benzamidenafil, Dasantafil, Dipyridamole, E4021, Icariin, Rolipram, Piclamilast, Tadalafil and Zaprinast, and their pharmaceutically acceptable salts.

14. The pharmaceutical composition as claimed in any of Claims 9-13, **characterized in that** the Statin analogue is one selected from the group consisting of Atorvastatin, Cerivastatin, Fluvastatin, Lovastatin, Mevastatin, Pravastatin, Rosuvastatin, Simvastatin, Pravastatin acid, and their pharmaceutically acceptable salts.
